# EUROPEAN PATENT SPECIFICATION

(11) **EP 1 560 617 B1**
(45) Date of publication and mention of the grant of the patent: **19.03.2008**
(21) Application number: 03762959.9
(22) Date of filing: 08.07.2003
(51) Int. Cl.: A61M 5/162

(54) **A COUPLING COMPONENT FOR TRANSMITTING MEDICAL SUBSTANCES**
KUPPLUNGSMITTEL ZUR ÜBERTRAGUNG VON MEDIZINISCHEN SUBSTANZEN
MOYEN D'ACCOUPLEMENT POUR TRANSMISSION DE SUBSTANCES MEDICALES

(30) Priority: 09.07.2002 US 394288 P; 09.07.2002 SE 0202174
(43) Date of publication of application: 10.08.2005
(73) Proprietor: Carmel Pharma AB, 402 28 Göteborg (SE)
(72) Inventor: WALLEN, Claes, 51840 Sjömarken (SE)
(74) Representative: Hammond, Andrew David
(86) International application number: PCT/SE2003/001193
(87) International publication number: WO 2004/004806

(56) References cited:
- EP-A1- 0 285 424
- EP-A2- 0 376 629
- EP-A2- 1 060 730
- WO-A1-98/19724
- WO-A2-02/02048
- US-A- 1 844 342
- US-A- 2 010 417
- US-A- 3 822 700
- US-A- 5 041 105
- US-A1- 2002 082 586

## Description

### FIELD OF THE INVENTION AND PRIOR ART

The present invention relates to a coupling component for transmitting medical substances, comprising two channels for conveyance of medical substances in two substantially opposite directions and a means for releasable connection, to a second coupling component having a further channel, for creating a coupling as defined in claim 1. The invention also relates to a method for conveyance of medical substances to and from a container, in which method a coupling component having two channels for conveyance of medical substances in two substantially opposite directions be connected to a second coupling component of a container, which second coupling component has a further channel.

The invention may be applied in different situations when medical substances are to be handled, but hereinafter the particular, but not in any way limiting for the invention, fields of application constituting a means for administration of fluids to/from infusion bags, which is desired in medical treatment for instance, will be described for illuminating purposes.

Infusion bags are used for intravenous delivery of fluids and medically effective substances to human beings and animals. For this reason, the infusion bag is provided with an outlet through which fluid may flow to a component connected to the patient, such as a cannula or the like, and further into the body of the patient. When preparing the fluids which are to be administrated to the body from the infusion bag, a usual method is that medically effective substances are supplied to a pre-sealed infusion bag which is filled with a transport fluid, such as a sodium chloride solution or a glucose solution. The preparation is performed by injecting the medically effective substance via an inlet into the bag.

For accomplishing the desired transportations of fluid a combined inlet and outlet of the infusion bag together with a coupling device which is denoted "spike" are often used. The spike has a first pointed end by means of which a membrane arranged in a narrow passage of the infusion bag, constituting inlet/outlet of the infusion bag, may be penetrated so that the infusion bag be opened towards two channels arranged in the spike when the spike is introduced in the inlet/outlet of the infusion bag. One of the channels is intended for conveyance of fluid in a direction from the infusion bag towards the patient and the other channel is intended for injection of medical substances into the infusion bag. In the other end of the spike are members arranged at the mouths of the channels for connection to other components, such as flexible tubes for conveyance of the fluid further to the patient and cannulas for the injection of medical substances to the infusion bag.

However, it has appeared that during certain extreme conditions there is a risk that the spike, which by insertion in the above mentioned narrow passage of the infusion bag is relatively loosely interconnected to the infusion bag, may unintentionally come loose from the infusion bag if the equipment is handled carelessly or by carelessness in connection with other treatment of the patient. The system of spike and infusion bag is depended of the friction between the infusion bag and the spike to prevent the spike from coming loose from the infusion bag. Furthermore, the spike has the disadvantage that leakage from the infusion bag to the environment may occur when the spike is introduced and the membrane is penetrated. In some cases the fluids which are to be administrated to the patient may be harmful to other persons than the patient who has been prescribed the treatment as a result of an indication of a specific decease. This is particularly the case when repeated long-term exposure is concerned, which can happened to medical staff when preparing and connecting infusion bags every day if the requisite security regulations are not fulfilled. A further disadvantage with the use of a spike which during the connection penetrates a membrane of the infusion bag for providing the fluid administration channels is that the connection step itself cannot be made in advance to later on enable conveyance of fluid from the infusion bag to a receiving unit connected to the spike, but the channels have to be opened instantaneously at the connecting moment.

US patent no. 3822700 discloses a piercing device provided with an unrestricted passage therethrough. When the device pierces a stopper of a bottle containing intravenous solution, additives may be injected into the bottle or a portion of the liquid in the bottle may be withdrawn through the unrestricted passage. The unrestricted passage may be opened by removing the cap or by piercing a diaphragm with a needle.

US patent no. 2010417 concerns a liquid flow apparatus for the administration of fluids, comprising a liquid container and a dispensing member. An adjustable liquid flow tube leads from a desired point in the liquid container to the dispensing member and an inlet tube is arranged within the liquid flow tube. The inlet tube is adjustable in relation to said liquid flow tube and leads from a desired point in the liquid container to the atmosphere or to a gas supply.

US patent no. 5041105A describes a connection component suitable for use with an enteral fluid delivery set which is arranged in the form of a replacement for the cap of a pre-filled, foil sealed fluid container. The connection component includes a fluid outlet passage which is arranged in the form of a projecting spike which penetrates and opens the foil seal as the connection component is attached to the pre-filled container.

European patent no. 376629 discloses a connection component suitable for use with an enteral fluid delivery set. The connection component consists of a threaded cap having a projecting spike thereon to penetrate and deform a foil diaphragm on the fluid container and further including an air passageway having a flexible member therein to allow filtered air to flow into the fluid container while preventing the flow of fluid from the fluid container through the air passageway.

International publication no. WO 9819724 concerns a device for administrating a toxic fluid, comprising an infusion device for connection to an infusion bag. The infusion device is provided with an insertion portion for connecting the bag, and an infusion chamber for dosing a fluid flow via a flow duct in the insertion portion from the bag to an outlet arranged on the chamber. The insertion portion also comprises a ventilating duct which extends between the bag and the outside of the infusion device and ends in a connection arranged on the side of the infusion device for supplying fluid to be administrated. The connection is provided with at least one membrane, which is air tight and penetrable by an injection needle.

### THE OBJECT OF THE INVENTION AND SUMMARY OF THE INVENTION

One object of the invention is to provide a coupling component/coupling of the kind defined by way of introduction for transmitting medical substances, in which coupling component/coupling at least some of the discussed disadvantages of such previously known coupling devices has been reduced to a great extent.

This object is achieved by providing a coupling component according to claim 1 and a coupling according to claim 8. By a coupling component/coupling having the feature that the connecting means is a thread/a thread joint it is ensured that the coupling not be unintentionally uncoupled when the coupling is tension loaded. By means of the thread/thread joint a coupling safe against tension load may be obtained at the same time as the connection may be accomplished quickly and safely in one simple operation. This implies that in the use of a device according to the invention, when an infusion bag is connected to a patient, an increased safety to the patient may be achieved at the same time as it is possible to deliver medical substances to the infusion bag and intravenously administrate fluid to the patient from the infusion bag. Furthermore the invention enables the use of other means, such as breakable fluid barrier plugs, for opening the infusion bag towards the channels and there is possible to ensure that leakage to the environment is prevented by tightening the thread joint before the infusion bag be opened towards the channels.

A further object of the invention is to provide a method of the kind defined by way of introduction, in which method a container and the coupling component according to claim 1, having two channels for conveyance of medical substances in two substantially opposite directions, may be connected to each other for conveyance of medical substances via the channels without the container being instantaneously opened towards the two channels.

This object is achieved by providing a method according to claim 10. Hereby the connection may be accomplished so as to later on enable conveyance of a medical substance from the container via one first of said two channels and/or conveyance of a medical substance to the container via the other of said two channels.

### SHORT DESCRIPTION OF THE DRAWINGS

A description in greater detail of exemplifying embodiments of the invention will follow below with reference to the attached drawings.

In the drawings:
Fig. 1 is a sectional view of an infusion bag and a spike according to prior art connected to the infusion bag,
Fig. 2 is a sectional view of an infusion bag and a coupling according to the invention for transmitting medical substances, and
Fig. 3 is a sectional view illustrating a variant of the coupling according to invention in Fig. 2.

### DETAILED DESCRIPTION OF PREFERRED EMBODIMENTS OF THE INVENTION

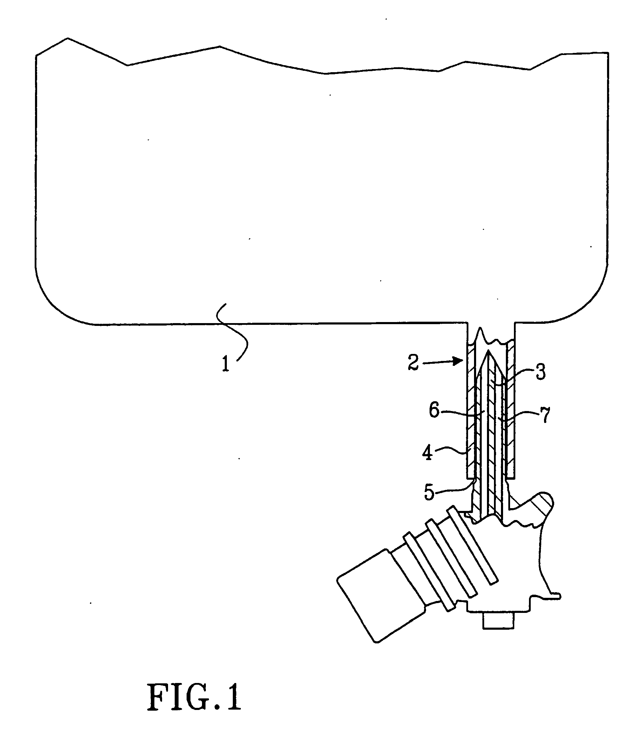

In Fig. 1 an infusion bag 1 and a coupling device 2 of the type called spike according to prior art are illustrated. The coupling device 2 is provided with a pointed part 3 which is introducible in a part 4 which constitutes an inlet/outlet of the infusion bag 1. This part 4 of the infusion bag 1 is designed as a flexible tube with a circular cross-section corresponding to the cross-section of the pointed part 3 of the spike and has, before the introduction of the pointed part of the spike, a sealing membrane arranged at the mouth 5 thereof. In the introduction of the spike the pointed part 3 penetrates the membrane and the infusion bag 1 be opened towards two channels 6, 7 arranged in the spike. Thereafter, medical substances may be injected into the infusion bag 1 via one first 6 of the channels and fluid may be transported out from the bag via the other 7 of the channels to a receiving unit (not illustrated).

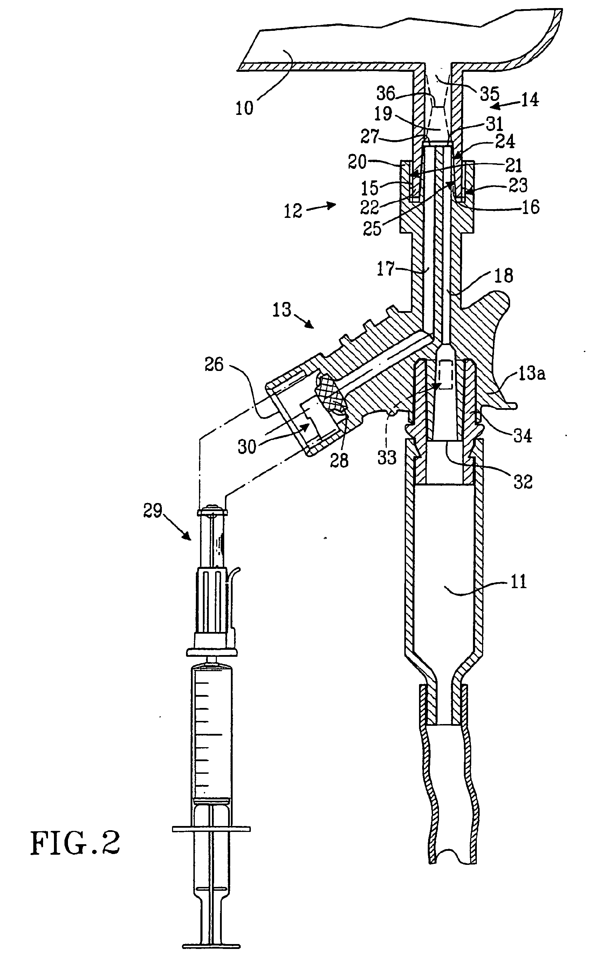

In Fig. 2 an infusion bag 10, a receiving unit 11 connected to the infusion bag and a coupling 12 according to the invention are illustrated. The coupling 12 comprises a first component 13, a second component 14 and a means 15 for releasable connection of the first 13 and second 14 components to each other for creating the coupling 12. The first component 13 comprises a mate fitting 16 provided with two channels 17, 18 for conveyance of medical substances in two substantially opposite directions. One first channel 17 for injecting medical substances into the infusion bag 10 and one second channel 18 for conveyance of fluid out of the infusion bag 10 to the receiving unit 11, which may be for example a chamber or the like for intravenous treatment. The second component 14 is provided with a further channel 19 intended to be in communication with said two channels 17, 18 of the first component 13.

The connecting means 15 is a thread joint having the characteristic that said first 13 and second 14 components are locked against rectilinear movement relative to each other when being connected to each other and the coupling 12 is tension loaded. The first component 13 has a ring 20 partly enclosing the male fitting 16, which ring has a internal thread 21 constituting part of the thread joint. The ring 20 is concentrically arranged relative to the male fitting 16. The second component 14 comprises a female fitting 22 provided with said further channel 19 and an external thread 23 corresponding to said thread 21 of the ring 20 and constituting part of the thread joint. When the first and second components are to be connected to each other, i.e. when the first and the second components be screwed together, the male fitting 16 be introduced into the female fitting 22 to form a connection between said two channels 17, 18 of the first component 13 and the further channel 19 of the second component 14, which connection is sealed relative to the environment. For this purpose the male fitting 16 and/or female fitting 22 may be designed with a certain taper so that when the male fitting and the female fitting have been brought together a certain distance the outer surface 24 of the male fitting will abut against the inner surface 25 of the female fitting, and then further movement of the components in the introduction direction relative to each other is not longer possible and a sealing between the male fitting 16 and the female fitting 22 is obtained when tightening the thread joint 15.

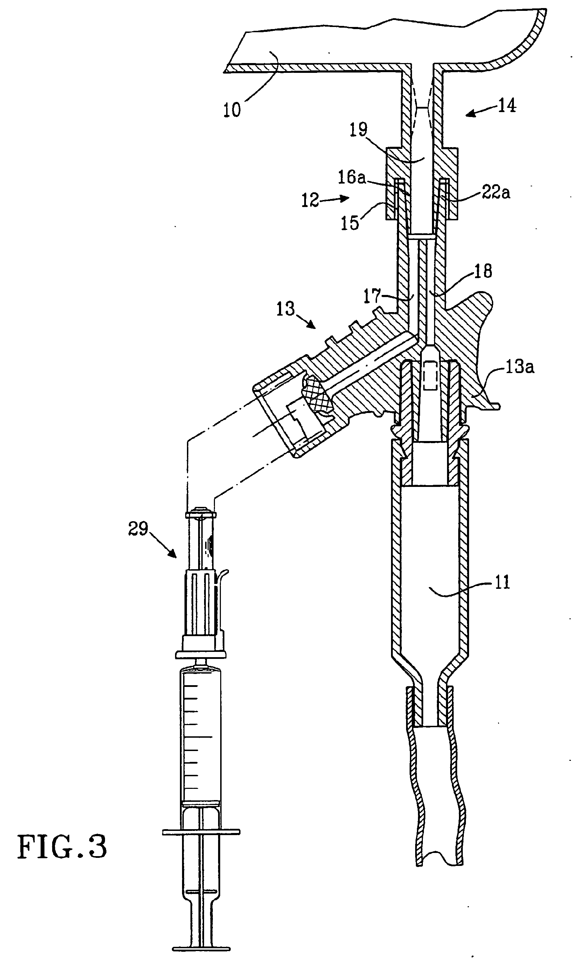

Although the coupling component according to the invention, i.e. said first component, which has the both channels, is designed as a male fitting of the coupling in the illustrated example, in another embodiment it could be designed as a female fitting 22a of the coupling 12, such as illustrated in Fig. 3, and the other component, which is arranged on for example an infusion bag, would in such a case be designed as a corresponding male fitting 16a. Of course it is also possible to change places of the threads in comparison to the illustrated embodiment in Fig. 2, so that instead, the second component including the female fitting is provided with an internal thread and the first component including the male fitting is provided with an external thread. The threads may for example be arranged on rings such as described above.

Advantageously the design of the threads 21, 23, the male fitting 16 and the female fitting 22 may be in accordance with a so called luer fitting coupling such as in the illustrated embodiments.

The coupling component 13 according to the invention is provided with a port 26 for injection of a medical substance to the first of the channels 17 and further conveyance of this substance to the infusion bag 10. For this purpose, the first channel 17 has also an outlet 27 arranged at one end of the first coupling component 13 which exhibits the connecting means 15 and the second coupling component 14 has said further channel 19, which communicates with the first 17 and the second 18 channels, for introduction or removal of liquid to/from the infusion bag 10.

Within the frame of the invention the injection port 26 may be designed in different ways depending on which injection component is to be connected. In the illustrated embodiment the injection port 26 has a first flexible membrane 28 for co-operation with a second flexible membrane (not illustrated) arranged in an injection component 29 which is connectable to the injection port 26. The first membrane 28 is suitably air- and liquid proof and penetratable by an injection needle. To achieve a sealed connection of such a injection component 29 to the injection port 26, the injection port has a means 30 for holding said second flexible membrane with a pressure against said first flexible membrane 28. This holding means 30 may for example be constituted by a snap lock device, bayonet coupling or similar. The current pressure in question may suitably be chosen so that said first and second membranes are pressed together to a pressure exceeding the yield point of the both membranes, which implies that fluid cannot be pressed out through the contact surfaces of the membranes and a sealed connection is obtained.

If a pressure exceeding the yield point is applied the membranes will exhibit same properties at the compressed surfaces as in an arbitrary cross-section through the membranes, which implies that liquid cannot be pressed through the contact surfaces of the membranes. Such a characteristic may be obtained when the said first and second membranes has been pressed together to a pressure exceeding 150 kPa. Since the device risks to be destroyed if it is subjected to exceedingly large contact forces, the contact pressure should be restricted as much as possible. It has been proved in experiments that the sufficient sealing without any risk of failure is obtained with contact forces of up to 11, 1N, which corresponds to 656 kPA. Preferably, the contact pressure is within the interval 300-473 kPa.

At one end of the first coupling component 13, which end exhibits the connecting means 15 for establishing communication with the further channel 19 of the second coupling component 14 of the infusion bag 10, the second channel 18 has a inlet 31. Furthermore, the first coupling component 13 has in the other end thereof a port 32 which works as an outlet for the second channel 18. Within the frame of the invention the outlet port 32 may be designed in several different ways depending on which unit is to be connected to the outlet port. For example a snap coupling 33 may be used in combination with a friction joint for retaining a connection unit at the outlet port. In accordance with an variant thereof the entire coupling component 13, or at least the part 13a closest to the outlet port 32a of the coupling component 13, may be made of a first material and the connection unit 34 corresponding to the outlet port may be made of a second material. In this connection, materials having considerably different elasticity may be chosen, preferably so that the second material has a considerable higher elasticity than the first material for providing sufficient sealing action between the coupling component 13 and the connection unit 34 and at the same time achieve that the coupling component 13 having a lower elasticity has a high resistance against deformation.

In the extension of the further channel 19, i.e. in a combined inlet and outlet 35 of the infusion bag 10 a breakable fluid barrier plug 36 is arranged. In a state of not has been broken the fluid barrier plug 36 prevents in-flowing and out-flowing via the combined inlet and outlet 35 of the infusion bag 10 which implies that the infusion bag 10 is sealed. After connecting the infusion bag 10 to the coupling component 13 according to the invention, and eventually to other components, the fluid barrier plug 36 may be broken so that the combined inlet and outlet 35 of the infusion bag 10 be opened towards the both channels 17, 18 in the coupling component 13 according to the invention.

The method according to the invention comprises connecting of the coupling component according to an embodiment of the invention 13 having two channels 17, 18 for conveyance of medical substances in two substantially opposite directions to a second coupling component 14 according to an embodiment of the invention belonging to a container 10, such as a infusion bag 10, which second coupling component has a further channel 19. After the coupling component 13 and the container 10 have been connected to each other, the container 10 be opened by means of a member 36, preferably in the form of a breakable fluid barrier plug or similar, which member is suitably arranged in a combine inlet and outlet 35 of the container 10, towards the both channels 17, 18 for enabling transportation of a medical substance to the container 10 via one first 17 of said two channels, and for enabling transportation of a medical substances from the container 10.

Preferably, the first 13 and the second 14 coupling components are connected to each other by a thread joint 15. By means of the thread joint 15 a male fitting 16 of the first component 13 may be brought into contact with a corresponding female fitting 22 of the second component 14 to form a connection sealed relative to the environment between said two channels 17, 18 and the further channel 19 of the second coupling component 14.

Then, the container 10 be opened towards 17, 18 by breaking the breakable fluid barrier plug 36. Thereafter, a medical substance may be injected to the container via one first 17 of said channels. Advantageously, the medical substance is injected by means of a component via a port arranged in the first coupling component which port has a first flexible membrane for co-operation with a second flexible membrane arranged in the injection component. Preferably, said second flexible membrane is held against said first flexible membrane with a pressure during the injection to prevent leakage and wastage during the injection. It has been proved that by holding said second flexible membrane against said first flexible membrane with a pressure exceeding the yield point of the first and second membranes it is ensured that the membranes fit tightly to each other in such away that fluid transportation between these membranes is prevented and thereby leakage to the environment is avoided. Parallel with the injection, the liquid state medical substance in the container may be transported via the second 18 of said two channels to a receiving unit.

It is stressed that the invention is not restricted to the exemplifying embodiments; rather within the scope of protection defined by the following claims, the invention may be varied in several ways once the idea of the invention is disclosed.

## Claims

1. A coupling (12) for transmitting medical fluids, comprising a first coupling component (13) having two channels (17, 18) each being for conveyance of medical fluids in two substantially opposite directions, a second coupling component (14) having a further channel (19), and a means (15) for releasable connection of the first and the second coupling components to each other for creating the coupling (12),
**characterised in that** the connection means is a thread joint (15) and that said further channel (19) is provided with a breakable fluid barrier plug (36).

2. A coupling according to claim 1, **characterized in that** the first coupling component (13) comprises male fitting (16) provided with said two channels (17, 18) and the second coupling component comprises a corresponding female fitting (22) provided with said further channel (19), to form a connection sealed relative to the environment between said two channels (17, 18) and said further channel (19) when the first and the second coupling components are connected to each other.

3. A coupling according to claim 2, **characterized in that** the first coupling component (13) comprises a ring (20) which is concentrically arranged relative to the male fitting (16) and at least partly encloses the male fitting, which ring is provided with a first thread (21) constituting part of the thread joint (15), and **in that** the female fitting (22) is provided with a second thread (23) corresponding to said first thread (21) and constituting part of the thread joint (15).

4. A. coupling according to any of claims 1-3, **characterized in that** the first coupling component (13) is provided with an opening (26) for, injection of a medical substance to a first (17) of said two channels.

5. A coupling according to claim 4, **characterized in that** the injection opening (26) has a first flexible membrane (28) for cooperation with a second flexible membrane arranged in an injection component (29) which is connectable to the injection opening (26).

6. A coupling according to claim 5, **characterized in that** it has a means (30) for holding said second flexible membrane with a pressure against said first membrane (28).

7. A coupling according to claim 6, **characterized in that** the pressure exceeds 150 kPa.

8. An infusion bag (10) comprising a coupling according to any of claims 1-7.

9. An infusion arrangement comprising an infusion bag (10) provided with a coupling (12) according to any of claims 1-7, for transmitting a medical fluid from an injection component to the infusion bag via one first of the channels and for transmitting a medical fluid from the infusion bag to a receiving unit connected to the coupling via the other of the channels.

10. A method for conveyance of medical fluids to a infusion bag (10), comprising connecting the first coupling component (13) of claim 1 having two channels (17, 18) for conveyance of medical fluids in two substantially opposite directions to the second coupling component (14) of claim 1 belonging to an infusion bag (10), said second coupling component having a further channel (19),
**characterized by** connecting the first coupling component (13) and the second coupling component (14) of the infusion bag (10) by means of a thread joint (15), and opening the infusion bag (10) towards said two channels (17, 18) after that the first coupling component (13) and the infusion bag (10) have been connected to each other, for enabling conveyance of a medical fluid into the infusion bag (10) via one first (17) of said two channels.

11. A method according to claim 10, **characterized by** opening the infusion bag (10) towards said two channels (17, 18) by breaking a breakable fluid barrier plug (36).

12. A method according to claim 10 or 11, **characterized by** opening the container (10) towards said two channels (17, 18) by means of a member (36) arranged in the infusion bag (10).

13. A method according to claim 10-12, **characterized by** injecting the medical fluid to the container (10) via one first (17) of said two channels.

14. A method according to claim 13, **characterized by** injecting the medical fluid by means of a component (29) via an opening (26) arranged in the first coupling component (13) which opening has a first flexible membrane (28) for cooperation with a second flexible membrane arranged in the injection component (29).

15. A method according to claim 14, **characterized by** holding said second flexible membrane against said first flexible membrane (28) with a pressure when injection is performed.

16. A method according to claim 15, **characterized by** holding said second flexible membrane against said first flexible membrane (28) with a pressure exceeding 150 kPa.

## Patentansprüche

1. Kupplung (12) zur Überleitung von medizinischen Flüssigkeiten, die ein erstes Kupplungsteil (13) mit zwei Kanälen (17, 18) zur Durchleitung von medizinischen Flüssigkeiten in zwei im Wesentlichen entgegen gesetzte Richtungen, ein zweites Kupplungsteil (14) mit einem weiteren Kanal (19) und eine Einrichtung (15) zur lösbaren Verbindung des ersten mit dem zweiten Kupplungsteil aufweist, um die Kupplung (12) herzustellen, wobei die Verbindungseinrichtung eine Schraubverbindung (15) ist, aufweist,
**dadurch gekennzeichnet, dass** der weitere Kanal (19) mit einem brechbaren Verschlussstopfen für Flüssigkeiten (36) versehen ist, der so gestaltet ist, dass er während der Verbindung des ersten Kupplungsteils (13) mit dem zweiten Kupplungsteil (14) unversehrt bleibt.

2. Kupplung gemäß Anspruch 1, **dadurch gekennzeichnet, dass** das erste Kupplungsteil (13) ein Anschlussstift (16) aufweist, der mit den zwei Kanälen (17, 18) versehen ist, und dass das zweite Kupplungsteil eine entsprechende Anschlussbuchse (22) aufweist, die mit dem weiteren Kanal (19) versehen ist, um, wenn das erste und das zweite Kupplungsteil miteinander verbunden werden, eine gegenüber der Umgebung abgedichtete Verbindung zwischen den beiden Kanälen (17,18) und dem weiteren Kanal (19) auszubilden.

3. Kupplung gemäß Anspruch 2, **dadurch gekennzeichnet, dass** das erste Kupplungsteil (13) einen Ring (20) aufweist, der in Bezug auf den Anschlussstift (16) konzentrisch angeordnet ist und den Anschlussstift zumindest teilweise umgibt, wobei der Ring mit einem ersten Gewinde (21) versehen ist, das einen Teil der Schraubverbindung (15) bildet, und dass die Anschlussbuchse (22) mit einem zweiten Gewinde (23) versehen ist, das dem ersten Gewinde (21) entspricht und einen Teil der Schraubverbindung (15) bildet.

4. Kupplung gemäß einem der Ansprüche 1-3, **dadurch gekennzeichnet, dass** das erste Kupplungsteil (13) mit einer Öffnung (26) zur Injektion einer medizinischen Substanz in einen ersten (17) der beiden Kanäle versehen ist.

5. Kupplung gemäß Anspruch 4, **dadurch gekennzeichnet, dass** die Injektionsöffnung (26) eine erste biegsame Membran (28) zum Zusammenwirken mit einer zweiten innerhalb eines Injektionsteils (29) angeordneten biegsamen Membran aufweist, die mit der Injektionsöffnung (26) verbunden werden kann.

6. Kupplung gemäß Anspruch 5, **dadurch gekennzeichnet, dass** sie eine Einrichtung zur Halterung der zweiten biegsamen Membran unter Druck gegen die erste Membran (28) aufweist.

7. Kupplung gemäß Anspruch 6, **dadurch gekennzeichnet, dass** der Druck 150kPa überschreitet.

8. Infusionsbeutel (10), der eine Kupplung gemäß einem der Ansprüche 1-7 aufweist.

9. Infusionsanordnung, die einen Infusionsbeutel (10) aufweist, der mit einer Kupplung (12) gemäß einem der Ansprüche 1-7 versehen ist, um eine medizinische Flüssigkeit von einem Injektionsteil zum Infusionsbeutel durch einen ersten der Kanäle überzuleiten und um eine medizinische Flüssigkeit vom Infusionsbeutel zu einer Empfangseinheit überzuleiten, die mit der Kupplung über den anderen der Kanäle verbundenen ist.

10. Verfahren zur Durchleitung von medizinischen Flüssigkeiten zu einem Infusionsbeutel (10), aufweisend ein Verbinden des ersten Kupplungsteils (13) gemäß Anspruch 1, welches zwei Kanäle (17,18) zur Durchleitung medizinischer Flüssigkeiten in zwei im Wesentlichen entgegen gesetzte Richtungen aufweist, mit dem zweiten Kupplungsteil (14) gemäß Anspruch 1, welches zu einem Infusionsbeutel (10) gehört, wobei das zweite Kupplungsteil einen weiteren Kanal (19) aufweist,
**dadurch gekennzeichnet, dass** das erste Kupplungsteil (13) und das zweite Kupplungsteil (14) des Infusionsbeutels (10) mittels einer Verschraubung (15) miteinander verbunden werden, und dass der Infusionsbeutel (10) zu den beiden Kanälen (17, 18) hin geöffnet wird, nachdem das erste Kupplungsteil (13) und der Infusionsbeutel (10) miteinander verbunden worden sind, um Durchleitung einer medizinischen Flüssigkeit über einen ersten (17) der beiden Kanäle in den Infusionsbeutel (10) zu ermöglichen.

11. Verfahren gemäß Anspruch 10, **dadurch gekennzeichnet, dass** der Infusionsbeutel (10) zu den zwei Kanälen (17,18) hin durch Brechen eines brechbaren Verschlussstopfens für Flüssigkeiten (36) geöffnet wird.

12. Verfahren gemäß Anspruch 10 oder 11, **dadurch gekennzeichnet, dass** der Behälter (10) mittels eines innerhalb des Infusionsbeutels (10) angeordneten Bauteils (36) zu den zwei Kanälen (17,18) hin geöffnet wird.

13. Verfahren gemäß Anspruch 10-12, **dadurch gekennzeichnet, dass** die medizinische Flüssigkeit durch einen ersten (17) der beiden Kanäle in den Behälter (10) injiziert wird.

14. Verfahren gemäß Anspruch 13, **dadurch gekennzeichnet, dass** die medizinische Flüssigkeit mittels eines Bauteils (29) durch eine innerhalb des ersten Kupplungsteils (13) angeordnete Öffnung (26) injiziert wird, wobei diese Öffnung eine erste biegsame Membran (28) aufweist, die mit einer zweiten, innerhalb des Injektionsteils (29) angeordneten, biegsamen Membran zusammenwirkt.

15. Verfahren gemäß Anspruch 14, **dadurch gekennzeichnet, dass** bei Durchführung einer Injektion die zweite biegsame Membran unter Druck gegen die erste biegsame Membran (28) gehalten wird.

16. Ein Verfahren gemäß Anspruch 15, **dadurch gekennzeichnet, dass** die zweite biegsame Membran gegen die erste biegsame Membran (28) unter einem Druck gehalten wird, der 150kPa überschreitet.

## Revendications

1. Moyens d'accouplement (12) pour transmission de fluides médicaux, comprenant un premier composant d'accouplement (13) ayant deux canaux (17, 18) pour transport de fluides médicaux dans deux directions sensiblement opposées, un deuxième composant d'accouplement (14) ayant un autre canal (19), et un moyen (15) pour connexion démontable des premier et deuxième composants d'accouplement l'un à l'autre, afin de créer l'accouplement (12), le moyen de connexion étant un joint vissé (15), **caractérisés en ce que** le dit autre canal (19) comporte un bouchon cassable d'arrêt des fluides (36) qui est agencé de manière à rester intact pendant la connexion du premier composant d'accouplement (13) au deuxième composant d'accouplement (14).

2. Moyens d'accouplement selon la revendication 1, **caractérisés en ce que** le premier composant d'accouplement (13) comprend un raccord mâle (16) comportant les dits deux canaux (17, 18) et le deuxième composant d'accouplement comprend un raccord femelle correspondant (22) comportant le dit autre canal (19), pour former une connexion étanche par rapport à l'environnement entre les dits deux canaux (17, 18) et le dit autre canal (19) lorsque le premier et le deuxième composants d'accouplement sont connectés l'un à l'autre.

3. Moyens d'accouplement selon la revendication 2, **caractérisés en ce que** le premier composant d'accouplement (13) comprend un anneau (20) qui est agencé concentriquement par rapport au raccord mâle (16) et entoure au moins en partie le raccord mâle, le dit anneau comportant un premier filetage (21) qui constitue une partie du joint fileté (15) et **en ce que** le raccord femelle (22) comporte un deuxième filetage (23) correspondant au dit premier filetage (21) et constituant une partie du joint vissé (15).

4. Moyens d'accouplement selon une quelconque des revendications 1 à 3, **caractérisés en ce que** le premier composant d'accouplement (13) comporte un orifice (26) pour injection d'une substance médicale dans un premier (17) des dits deux canaux.

5. Moyens d'accouplement selon la revendication 4, **caractérisés en ce que** l'orifice d'injection (26) comprend une première membrane flexible (28) pour coopération avec une deuxième membrane flexible agencée dans un composant d'injection (29) qui est connectable à l'orifice d'injection (25).

6. Moyens d'accouplement selon la revendication 5, **caractérisés en ce qu'**un moyen (30) est prévu pour appliquer la dite deuxième membrane flexible avec une certaine pression contre la dite première membrane (28).

7. Moyens d'accouplement selon la revendication 6, **caractérisés en ce que** la pression est supérieure à 150 kPa.

8. Poche de perfusion (10) comprenant des moyens d'accouplement selon une quelconque des revendications 1 à 7.

9. Dispositif de perfusion comprenant une poche de perfusion (10) pourvue de moyens d'accouplement (12) selon une quelconque des revendications 1 à 7, pour transmettre un fluide médical d'un composant d'injection à la poche de perfusion via un premier des canaux, et pour transmettre un fluide médical de la poche de perfusion à une unité de réception connectée aux moyens d'accouplement via l'autre des canaux.

10. Procédé pour le transport de fluides médicaux à une poche de perfusion (10), comprenant la connexion du premier composant d'accouplement (13) de la revendication 1, ayant deux canaux (17, 18) pour transport des fluides médicaux dans deux directions sensiblement opposées, au deuxième composant d'accouplement (14) de la revendication 1 appartenant à une poche de perfusion (10), le dit deuxième composant d'accouplement ayant un autre canal (19),
**caractérisé par** la connexion du premier composant d'accouplement (13) et du deuxième composant d'accouplement (14) de la poche de perfusion (10) au moyen d'un joint vissé (15), et l'ouverture de la poche de perfusion (10) vers les dits deux canaux (17, 18) après que le premier composant d'accouplement (13) et la poche de perfusion (10) aient été connectés l'un à l'autre, pour permettre le transport d'un fluide médical vers la poche de perfusion (10) via un premier (17) des dits deux canaux.

11. Procédé selon la revendication 10, **caractérisé par** l'ouverture de la poche de perfusion (10) vers les dits deux canaux (17, 18) par cassure d'un bouchon cassable d'arrêt des fluides (36).

12. Procédé selon la revendication 10 ou 11, **caractérisé par** l'ouverture du récipient (10) vers les dits deux canaux (17, 18) au moyen d'un élément (36) disposé dans la poche de perfusion (10).

13. Procédé selon la revendication 10 à 12, **caractérisé par** l'injection du fluide médical dans le récipient (10) via un premier (17) des dits deux canaux.

14. Procédé selon la revendication 13, **caractérisé par** l'injection du fluide médical au moyen d'un composant (29) via un orifice (26) prévu dans le premier composant d'accouplement (13), le dit orifice ayant une première membrane flexible (28) pour coopération avec une deuxième membrane flexible agencée dans le composant d'injection (29).

15. Procédé selon la revendication 14, **caractérisé par** le maintien de la dite deuxième membrane flexible contre la dite première membrane flexible (28) sous une certaine pression lorsque l'injection est effectuée.

16. Procédé selon la revendication 15, **caractérisé par** le maintien de la dite deuxième membrane flexible contre la dite première membrane flexible (28) sous une pression supérieure à 150 kPa.
